# EUROPEAN PATENT SPECIFICATION

(11) **EP 4 110 445 B1**
(45) Date of publication and mention of the grant of the patent: **16.04.2025**
(21) Application number: 21711480.0
(22) Date of filing: 26.02.2021
(51) Int. Cl.: A61M 16/06

(54) **SEALING CUSHION**
DICHTKISSEN
COUSSIN D'ÉTANCHÉITÉ

(30) Priority: 28.02.2020 GB 202002901
(43) Date of publication of application: 04.01.2023
(73) Proprietor: Intersurgical AG, 9490 Vaduz (LI)
(72) Inventor: BOWSHER, Richard Francis, Wokingham Berkshire RG41 2RZ (GB)
(74) Representative: Adamson Jones
(86) International application number: PCT/EP2021/054903
(87) International publication number: WO 2021/170835

(56) References cited:
- WO-A1-2006/074513
- WO-A1-2016/032343
- GB-A- 2 521 644

## Description

The present disclosure relates to sealing cushions for respiratory mask assemblies.

Non-invasive ventilation is a process by which a flow of respiratory gas is delivered to the airway of a patient through a non-invasive device, generally a respiratory mask, preventing the need for an invasive device such as an endotracheal tube that enters a patient's airway. Non-invasive ventilation is typically used to manage both chronic and acute respiratory failure, as well as other medical disorders such as sleep apnoea.

Leakage from a respiratory mask during use is undesirable because it reduces alveolar ventilation and synchrony between the patient and the ventilator, and can create unpleasant drafts, causing discomfort to the patient. As a result, respiratory masks for use in non-invasive ventilation are generally adapted to form an effective seal against the patient's face. For this reason, resiliently deformable sealing cushions are typically used to provide a patient interface portion of a respiratory mask.

Whilst such sealing cushions are able to resiliently deform to generally match the contours of a patient's face, there nevertheless remain gaps between the sealing cushion and the patient's face which allow leakage of the respiratory gas. In order to eliminate such leakage it is necessary to apply further force, usually through the use of an elasticated band or strap, to further engage the sealing cushion with a patient's face, thus eliminating any gaps in the seal. However, this can cause discomfort to a patient.

Furthermore, over deformation of the sealing cushion can cause unwanted stress in the material of the device, which may eventually lead to degradation and failure of the cushion, and may also result in improper location of the sealing cushion, allowing the sealing cushion to move from its desired location during use.

Patent document WO2016/032343A1 is hereby acknowledged.

There has now been devised an improved cushion for use with a respiratory mask assembly, which overcomes or substantially mitigates the aforementioned and/or other disadvantages associated with the prior art.

According to a first aspect of the disclosure not explicitly covered by the independent claims of this application, there is provided a sealing cushion for a respiratory mask assembly, the sealing cushion comprising a patient interface portion having a resiliently deformable sealing membrane for engagement with a patient's face, and an aperture formed therein for receiving a nasal and/or mouth region of a patient's face, the sealing membrane comprising a plurality of regions of differing thickness, the plurality of regions comprising: a compliant nasal region for engagement with the patient's nasal bridge; nasal support regions disposed either side of the compliant nasal region for engagement with the sides of the patient's nose; cheek support regions extending from each of the nasal support regions for engagement at least with the patient's cheeks; and a compliant base region extending between the cheek support regions, wherein the sealing membrane in the nasal support regions has a greater thickness than in the cheek support regions, and wherein the sealing membrane in the compliant nasal region and the compliant base region has a lower thickness than in the cheek support regions and the nasal support regions.

The sealing cushion according to the technology is principally advantageous in that it provides a configuration of regions of differing flexibility and/or resilience which have been found to provide an improved seal around the patient's face, whilst also providing improved comfort to the patient.

The patient interface portion may be shaped in the rest configuration to substantially match contours of a patient's face. The sealing membrane may present a convex contact surface to the patient's face. The sealing membrane may be curved in cross-section in the region of the patient interface portion. The curvature of the sealing membrane may increase on deformation caused by engagement with the patient's face.

The patient interface portion and the aperture therein may be substantially triangular in nature. The patient interface and the aperture therein may comprise an apex arranged to engage with a wearer's nasal region, and two base corners. The patient interface and the aperture therein may comprise a base edge and/or base region positioned between the base corners. That is, the shape of the aperture may correspond to the shape of the patient interface portion. The aperture may be disposed substantially centrally within the sealing membrane. The aperture may conform substantially to the shape of a nasal and/or mouth region of the patient. The corners of the aperture may be rounded or curved. The sealing membrane in the region of the aperture may curve slightly inwardly towards an internal cavity of the sealing cushion.

The sealing cushion may comprise a mask connection interface for connection to a respiratory mask body. The mask interface portion may be, and is most preferably, disposed on an opposing side of the sealing cushion to the patient interface portion. The sealing cushion may comprise a sidewall portion extending between the mask connection interface and the patient interface portion.

The patient interface portion and the sidewall portion may be formed as one continuous membrane. The continuous membrane may extend from the mask connection interface to form the sidewall portion. The continuous membrane may be folded upon itself towards the centre of the sealing cushion to form the patient interface portion such that the patient interface portion is substantially perpendicular to the sidewall portion. That is, the continuous membrane may be curved in cross-section between the side wall portion and the patient interface portion.

The membrane may be formed of a thermoplastic elastomer (TPE) or a thermoset elastomer (TSE), such as silicone. The membrane may comprise a single membrane. For example, the membrane may have no underlying support member and/or cushion, such that the only resistance to deformation is provided by the membrane and associated regions of differing thickness.

The thickness of each region may vary across the region such that the transitions between regions of differing thickness may be gradual transitions. Alternatively, the thickness of each region may be constant across the region such that the transitions between regions of differing thickness may be distinct transitions, eg steps, or shoulders.

The compliant nasal region may be positioned at the apex of the patient interface portion. The compliant nasal region may be positioned and arranged so as to extend over the nasal bridge of a patient in use.

The nasal support regions may comprise a first nasal support region disposed to the left of the compliant nasal region and a second nasal support region disposed to the right of the compliant nasal region, when viewing the patient interface portion.

The cheek support regions may comprise a first cheek support region extending from the first nasal support region and a second cheek support region extending from the second nasal support region. The majority of the cheek support regions may be disposed closer to the base corners of the patient interface portion relative to the majority of the nasal support regions. There may be some overlap between a lower portion of the nasal support regions and an upper portion of the cheek support regions.

The compliant base region may be disposed in the base region of the patient interface portion. The compliant base region may be disposed along the base edge of the patient interface portion. The compliant base region may be disposed in the central region of the base region of the patient interface portion.

Each cheek support region may comprise a reinforced subregion. The reinforced subregion may be a subregion of the cheek support region that has a greater thickness than the remainder of the cheek support region, or at least the majority of the remainder of the cheek support region. The reinforced subregion may be separated from the nasal support regions by a region of different thickness. For example, the reinforced subregion may be separated from the nasal support regions by a portion of the cheek support region. The reinforced subregions may comprise reinforced subregions disposed at each of the base corners of the patient interface portion.

This is advantageous in that the reinforced subregions provide additional rigidity in the positions of the sealing cushion that are most commonly held to apply force to improve the seal around the patient's nasal and/or mouth region in use.

The patient interface portion may further comprise a compliant inner member, which may define the boundary of the aperture in the patient interface portion. The compliant inner member may extend around substantially all of the perimeter of the aperture in the patient interface portion. Preferably, the compliant inner member may extend around the entire perimeter of the aperture in the patient interface portion. The compliant inner member may take substantially the same shape as the aperture in the patient interface portion. The compliant inner member may merge with the compliant nasal region at the apex of the aperture. The compliant inner member may merge with the compliant base region in the base region of the patient interface portion. The compliant inner member may have substantially the same thickness as the compliant nasal region and/or the compliant base region. The compliant inner member may have substantially the same width over the majority of its perimeter. The compliant inner member may be slightly wider in the regions adjacent the nasal support regions.

This is advantageous in that the compliant inner member is positioned around the edge of the aperture, which almost fully engages with the patient in use. This provides greater comfort to the patient upon engagement, since the compliant inner member provides greater flexibility than the majority of the sealing cushion. This greater flexibility also allows the sealing cushion to fit to the user better, thus enhancing the sealing properties of the cushion.

The patient interface portion may be symmetrical or substantially symmetrical about a median plane extending from the centre of the base region of the patient interface portion to the apex of the patient interface portion.

The sidewall portion may comprise reinforced sidewall regions. The reinforced sidewall regions may be subregions of the sidewall portion. The reinforced sidewall regions may comprise a first reinforced sidewall region disposed on the left-hand sidewall portion of the sealing cushion and a second reinforced sidewall region disposed on the right-hand sidewall portion of the sealing cushion.

The compliant nasal region may also extend across at least a portion of the sidewall portion. The compliant nasal region may comprise a narrow band extending substantially longitudinally from the compliant inner member of the patient interface portion to the apex of the patient interface portion. The compliant nasal region may increase in its width from the aperture in the patient interface portion to the apex of the patient interface portion. The increase in its width over the patient interface portion may be greater than its increase in width over the sidewall portion.

The nasal support regions may extend along the left and right sides of the patient interface portion, between the compliant nasal region and the cheek support regions. The nasal support regions may also extend across at least a portion of the sidewall portion, ie to form a reinforced subregion of the sidewall portion. The nasal support regions may comprise lobes that would be substantially ear-shaped, if the patient interface portion and the sidewall portion were to be laid flat.

The mask may be defined as having a median plane that spans through the mask from front to back along a longitudinal axis of the mask which runs between the apex and the centre of the base region, where the median plane corresponds to the median plane of a wearer when in use. The median plane of the mask may divide the mask into substantially symmetrical right-hand and left-hand halves.

The mask may be defined as having a transverse plane that spans through the mask from front to back, along a transverse axis of the mask, the transverse axis running between the left-hand side and right-hand side of the mask, where the transverse plane corresponds to the transverse plane of a wearer when in use. The median plane and the transverse plane are perpendicular planes.

Each nasal support region may comprise a leading edge that is substantially parallel to the median plane, which may also be substantially parallel to the longitudinal axis of the mask. Each nasal support region may be adjacent the compliant nasal region and/or the compliant inner member. The edge may therefore be substantially perpendicular to the transverse plane.

Where the nasal support regions extend across at least a portion of the sidewall portion, each nasal support region may comprise top and bottom edges that are substantially parallel to the median plane, which may be substantially perpendicular to longitudinal axis of the mask. Each nasal support region may be positioned on the sidewall portion and may be adjacent to the compliant nasal region. That is, the edges are substantially parallel to both the transverse plane and the median plane. Here, the top edge refers to the edge positioned closest to the apex, and the bottom edge refers to the edge positioned furthest from the apex.

At the bottom end of the leading edge defined above, the edge of each nasal support region may curve away from the aperture in the patient interface portion, towards the transition between the patient interface portion and the sidewall portion.

The cheek support regions may extend along the left and right sides of the patient interface portion, between the nasal support regions and the base corners of the patient interface portion. Where there is an overlap between a lower portion of the nasal support regions and an upper portion of the cheek support regions, the upper portions of the cheek support regions may extend alongside the nasal support region. The cheek support regions may further extend along the base edge of the patient interface portion, between the base corners of the patient interface portion and the compliant base region.

The cheek support regions may take substantially the same shape as the sealing cushion over the patient interface portion. That is, the cheek support regions may substantially follow the curvature of the patient interface portion. The cheek support regions may also extend across at least a portion of the sidewall portion, ie to form a reinforced subregion of the sidewall portion. The cheek support regions may extend along the left and right sides of the sidewall portion, between the nasal support regions and the lower left and right corners of the patient interface portion. The cheek support regions may further extend along the lower side of the sidewall portion, between the lower left and right corners of the patient interface portion and the compliant base region.

Generally, the cheek support regions may extend between the aperture in the patient interface portion and the reinforced sidewall portions. Where the patient interface portion comprises a compliant inner member, the cheek support regions may extend between the compliant inner member and the reinforced sidewall portions. At least one portion of each cheek support region may extend from the aperture or the compliant inner member of the patient interface portion across the sidewall portion to the mask connection interface. The at least a portion of each cheek support region may comprise the point at which the cheek support region meets the compliant base region. At least one portion of each cheek support region may extend between the aperture or the compliant inner member and the corresponding nasal support region.

The compliant base region may comprise a substantially rectangular shaped portion. The compliant base region may further extend across the sidewall portion. The compliant base region may extend from the aperture in the patient interface portion across the sidewall portion to the mask connection interface. Where the patient interface portion comprises a compliant inner member, the compliant base region may extend from the compliant inner member across the sidewall portion to the mask connection interface. In the compliant base region, the sealing membrane may be less curved in cross-section between the side wall portion and the patient interface portion than the rest of the sealing cushion, ie the angle of curvature between the sidewall portion and the patient interface portion in the compliant base region may be less than in the rest of the sealing cushion.

The reinforced subregions may comprise substantially square-shaped or rectangular-shaped portions, which may have rounded corners. The reinforced subregions may be disposed at the base corners of the patient interface portion, or proximal thereto. The reinforced subregions may extend from the patient interface portion across the sidewall portion. The reinforced subregions may extend from the lower left and right corners of the cheek support regions across the sidewall portion to the reinforced sidewall portions.

The reinforced sidewall portions may take substantially the same shape as the sealing cushion over the sidewall portion. That is, the reinforced sidewall portions may substantially follow the curvature of the sidewall portion. In this regard, the reinforced sidewall portions may extend over the sidewall portion substantially parallel to the cheek support regions. Each reinforced sidewall portion may extend across the sidewall portion between the mask connection interface and the cheek support region. Each reinforced sidewall portion may extend along the sidewall portion between the nasal support region and the cheek support region. The top of each reinforced sidewall portion may be disposed at approximately the same position as the lower point of the undulating edge of the nasal support region.

Where the respiratory mask assembly is a full-face respiratory mask assembly, the aperture formed in the sealing cushion may be for receiving a nasal and mouth region of the patient's face, and the following features may be present or applicable.

The compliant base region may be a compliant chin region positioned so as to engage with the upper chin of a patient in use. The reinforced subregions may comprise reinforced jaw portions positioned so as to engage with the jaw of a patient in use, ie below the lower lip.

The sidewall portion may comprise a nasal bridge support region. The nasal bridge support region may be disposed at the upper end of the sidewall portion, ie towards the apex. The nasal bridge support region may extend between the mask connection interface and the compliant nasal region. The nasal bridge support region may extend between the nasal support regions.

This is advantageous in that the nasal bridge support region provides additional rigidity in a position of the sealing cushion that is commonly held to apply force to improve the seal around the patient's nasal and/or mouth region in use.

The compliant nasal region may further extend over the sidewall portion between the apex of the patient interface portion and the nasal bridge support region. The compliant nasal region may increase in its width over the sidewall portion from the apex of the patient interface portion to the nasal bridge support region.

At least a portion of each nasal support region may extend from the aperture in the patient interface portion across the sidewall portion to the mask connection interface. Where the patient interface portion comprises a compliant inner member, at least a portion of each nasal support region may extend from the compliant inner member of the patient interface portion across the sidewall portion to the mask connection interface.

Where the edge of each nasal support region curves away from the aperture in the patient interface portion, towards the transition between the patient interface portion and the sidewall portion, this edge may comprise an undulating edge that extends between the leading edge and the bottom edge, as defined above.

Each nasal support region, at its widest point, may extend the full distance between the mask connection interface and the aperture in the patient interface portion. Where the patient interface portion comprises a compliant inner member, each nasal support region, at its widest point, may extend the full distance between the mask connection interface and the compliant inner member. The widest point of each nasal support region may be towards the upper end of the sealing cushion, ie towards the apex. Each nasal support region, at its narrowest point, may only extend between the mask connection interface and the transition between the patient interface portion and the sidewall portion.

At least one portion of each cheek support region may extend from the aperture or the compliant inner member of the patient interface portion across the sidewall portion to the mask connection interface. The at least a portion of each cheek support region may comprise the point at which the cheek support region meets the compliant base region. Where at least one portion of each cheek support region extends between the aperture or the compliant inner member and the corresponding nasal support region, in this portion, the upper edge of each cheek support region, ie the edge closest to the apex, may be an undulating edge that corresponds to the undulating edge of the corresponding nasal support region.

Where the reinforced sidewall portions substantially follow the curvature of the sidewall portion, the reinforced sidewall portions may extend over the sidewall portion substantially parallel to the cheek support regions. Each reinforced sidewall portion may extend along the sidewall portion between the nasal support region and the cheek support region. The top of each reinforced sidewall portion may be disposed at approximately the same position as the lower point of the undulating edge of the cheek support region.

The nasal bridge support region may comprise a narrow band having curved edges adjacent the mask connection interface and the compliant nasal region. The nasal bridge support region may have a greater width than it does length, when viewed from the direction of the apex of the sidewall portion. That is, the nasal bridge support region may extend further between the nasal support regions than it does between the mask connection interface and the compliant nasal region. The width of the nasal bridge support region, when viewed from the direction of the apex of the sidewall portion, may be substantially the same as the compliant nasal region. Alternatively, the width of the nasal bridge support region, when viewed from the direction of the apex of the sidewall portion, may be greater than the width of the compliant nasal region.

The compliant nasal region may be of a first thickness. The compliant base region may be of a second thickness. The compliant inner member may be of a third thickness. The cheek support regions may be of a fourth thickness. The reinforced subregions may be of a fifth thickness. The reinforced sidewall portions may be of a sixth thickness. The nasal bridge support region may be of a seventh thickness. The nasal support regions may be of an eighth thickness.

The first, second and third thicknesses may be equal or substantially equal, eg having less than 1% difference, having less than 2% difference, having less than 5% difference, or having less than 10% difference. The sixth and seventh thicknesses may be equal or substantially equal. The fourth thickness may be greater than the first, second and third thicknesses, but less than the fifth, sixth, seventh and eight thicknesses. The fifth thickness may be greater than the first, second, third and fourth thicknesses, but less than the sixth, seventh and eighth thicknesses. The eight thickness may be greater than the first, second, third, fourth, fifth, sixth and seventh thicknesses. Alternatively, each of the first, second, third, fourth, fifth, sixth, seventh and eight thicknesses may be different, and the order of thicknesses may be different, where the first, second and third thicknesses are less than all of the fourth, fifth, sixth, seventh and eight thicknesses.

The first, second and third thicknesses may be between approximately 0.2mm and approximately 0.4mm, for example between 0.25mm and 0.35mm. The fourth thickness may be between approximately 1mm and approximately 1.5mm, for example between 1.25mm and 1.45mm. The fifth thickness may be between approximately 1.7mm and approximately 2.2mm, for example between 1.85mm and 2.05mm. The sixth and seventh thicknesses may be between approximately 1.8mm and approximately 2.2 mm, for example between 1.9mm and 2.1mm. The eighth thickness may be between approximately 2.25mm and approximately 2.75mm, for example between 2.4mm and 2.6mm.

The sealing membrane may have a first angle of curvature between the sidewall portion and the patient interface portion in the compliant base region, a second angle of curvature in the compliant nasal region, a third angle of curvature in the region of the nasal support regions, a fourth angle of curvature in the region of the reinforced subregions, and a fifth angle of curvature in the region of the cheek support regions.

The first angle of curvature may be less than the second, third, fourth and fifth angles of curvature. The second and third angles of curvature may be equal or substantially equal, eg having less than 1% difference, having less than 2% difference, having less than 5% difference, or having less than 10% difference. The fourth and fifth angles of curvature may be less than the second and third angles of curvature. The fourth and fifth angles of curvature may be equal or substantially equal. Alternatively, each of the angles of curvature may be different, and the order of angles of curvature may be different, where the first angle of curvature is less than second, third, fourth and fifth angles of curvature.

Where the respiratory mask assembly is a nasal respiratory mask assembly, the aperture formed in the sealing cushion may be for receiving only a nasal region of the patient's face, and the following features may be present or applicable.

The compliant base region may be positioned so as to engage with the philtrum of a patient in use. That is, the nasal support regions and cheek support regions may be reduced in length in order to reduce the overall length of the sealing cushion. In this example, the length of the sealing cushion refers to the distance between the apex of the patient interface portion and the base edge of the patient interface portion.

The compliant nasal region may further extend over the sidewall portion between the apex of the patient interface portion and the mask connection interface. The compliant nasal region may increase in its width over the sidewall portion from the apex of the patient interface portion to the mask connection interface.

At least a portion of each nasal support region may extend from the aperture in the patient interface portion across the sidewall portion to the reinforced sidewall portion. Where the patient interface portion comprises a compliant inner member, at least a portion of each nasal support region may extend from the compliant inner member of the patient interface portion across the sidewall portion to the reinforced sidewall portion.

Where the nasal support regions extend across at least a portion of the sidewall portion, each nasal support region may comprise a leading edge that is obliquely angled relative to the median plane. Each nasal support region may comprise a leading edge that is angled relative to the longitudinal axis of the mask. Each nasal support region may be adjacent the compliant nasal region and/or the compliant inner member. The edge may therefore be obliquely angled relative to the transverse plane.

Each nasal support region may comprise top and bottom edges that are substantially parallel to the median plane, which may be substantially perpendicular to longitudinal axis of the mask. Each nasal support region may extend onto the sidewall portion and may be adjacent to the compliant nasal region. That is, the edges are substantially parallel to both the transverse plane and the median plane. Here, the top edge refers to the edge positioned closest to the apex, and the bottom edge refers to the edge positioned furthest from the apex.

Each nasal support region, at its widest point, may extend the full distance between the reinforced sidewall portion and the aperture in the patient interface portion. Where the patient interface portion comprises a compliant inner member, each nasal support region, at its widest point, may extend the full distance between the reinforced sidewall portion and the compliant inner member. The widest point of each nasal support region may be towards the upper end of the sealing cushion, ie towards the apex. Each nasal support region, at its narrowest point, may only extend between the reinforced sidewall portion and the transition between the patient interface portion and the sidewall portion.

At least one portion of each cheek support region may extend from the aperture or the compliant inner member of the patient interface portion across the sidewall portion to the reinforced sidewall portion. The at least a portion of each cheek support region may comprise the point at which the cheek support region meets the compliant base region.

Where at least one portion of each cheek support region extends between the aperture or the compliant inner member and the corresponding nasal support region, in this portion, a leading edge of each cheek support region may be substantially parallel to the leading edge of the corresponding nasal support region.

Each reinforced sidewall portion may extend along the sidewall portion between the compliant nasal region and the compliant base region. The top of each reinforced sidewall portion may be disposed at approximately the same position as the top of each nasal support region.

One or each nasal support region may comprise a reinforced nasal region.

The sidewall portion may further comprise at least one reinforcing member. The sidewall portion may comprise at least one reinforcing member disposed on the left-hand sidewall portion of the sealing cushion and at least one reinforcing member disposed on the right-hand sidewall portion of the sealing cushion. The sidewall portion may comprise at least one upper reinforcing member and at least one lower reinforcing member. The sidewall portion may comprise one upper reinforcing member disposed on the left-hand sidewall portion of the sealing cushion and one upper reinforcing member disposed on the right-hand sidewall portion of the sealing cushion. The sidewall portion may comprise one lower reinforcing member disposed on the left-hand sidewall portion of the sealing cushion and one lower reinforcing member disposed on the right-hand sidewall portion of the sealing cushion.

The at least one upper reinforcing member may extend across the reinforced sidewall region between the mask connection interface and the reinforced nasal region. The at least one lower reinforcing member may extend across the reinforced sidewall region between the mask connection interface and the reinforced subregion. The at least one upper reinforcing member and the at least one lower reinforcing member may be substantially rectangular portions. The at least one upper reinforcing member and the at least one lower reinforcing member may have a constant width across their length. The at least one upper reinforcing member may have a central axis running between the mask connection interface and the reinforced nasal region that is substantially parallel to the transverse plane and/or substantially parallel to the median plane. The at least one lower reinforcing member may have a central axis running between the mask connection interface and the reinforced subregion that is substantially parallel to the transverse plane and/or substantially parallel to the median plane.

The compliant nasal region may be of a first thickness. The compliant base region may be of a second thickness. The compliant inner member may be of a third thickness. The cheek support regions may be of a fourth thickness. The nasal support regions may be of a fifth thickness. The reinforced subregions may be of a sixth thickness. The reinforced sidewall portions may be of a seventh thickness. The reinforced nasal regions may be of an eight thickness. The upper reinforcing members may be of a ninth thickness. The lower reinforcing members may be of a tenth thickness.

The first, second and third thicknesses may be equal or substantially equal. The fourth thickness may be greater than the first, second and third thicknesses. The fifth thickness may be greater than the first, second, third and fourth thicknesses. The sixth thickness may be greater than the first, second, third, fourth and fifth thicknesses. The seventh and eighth thickness may be equal or substantially equal. The seventh and eighth thicknesses may be greater than the first, second, third, fourth, fifth, and sixth thicknesses. The ninth and tenth thicknesses may be equal or substantially equal. The ninth and tenth thicknesses may be greater than the first, second, third, fourth, fifth, sixth, seventh and eighth thicknesses. Substantially equal may mean having less than 1% difference, having less than 2% difference, having less than 5% difference, or having less than 10% difference.

Alternatively, each of the first, second, third, fourth, fifth, sixth, seventh, eighth, ninth and tenth thicknesses may be different, and the order of thicknesses may be different, where the first, second and third thicknesses are less than all of the fourth, fifth, sixth, seventh, eighth, ninth and tenth thicknesses.

The first, second and third thicknesses may be between approximately 0.2mm and approximately 0.4mm, for example between 0.25mm and 0.35mm. The fourth thickness may be between approximately 0.5mm and approximately 0.9mm, for example between 0.6mm and 0.8mm. The fifth thickness may be between approximately 0.8mm and approximately 1.2mm, for example between 0.9mm and 1.1mm. The sixth thickness may be between approximately 1mm and approximately 1.5 mm, for example between 1.15mm and 1.35mm. The seventh thickness may be between approximately 1.8mm and approximately 2.2mm, for example between 1.9mm and 2.1mm. The eighth thickness may be between approximately 1.7mm and approximately 2.1mm, for example between 1.8mm and 2.0mm. The ninth and tenth thicknesses may be between approximately 2mm and approximately 2.5mm, for example between 2.15mm and 2.35mm.

The sealing membrane may have a first angle of curvature between the sidewall portion and the patient interface portion in the compliant base region, a second angle of curvature in the compliant nasal region, a third angle of curvature in the region of the nasal support regions, and a fourth angle of curvature in the region of the reinforced subregions.

The first, second and third angles of curvature may be equal or substantially equal, eg having less than 1% difference, having less than 2% difference, having less than 5% difference, or having less than 10% difference. The first, second and third angles of curvature may be approximately 90 degrees, ie between 75 and 105 degrees or between 80 and 100 degrees. The fourth angle of curvature may be greater than the first, second and third angles of curvature, for example greater than 90 degrees. Alternatively, each of the angles of curvature may be different, and the order of angles of curvature may be different.

The following features may be present or applicable where the respiratory mask is either a full-face respiratory mask assembly or a nasal respiratory mask assembly.

The sealing cushion may be adapted to fasten, in a peripheral region, to a mask body, which may be more rigid than the sealing cushion and have the form of a mask shell. In particular, the mask connection interface may comprise a recess for receiving a corresponding projection of a mask shell, eg with a close fit and/or a snap fit. The recess may extend around substantially all of the perimeter of the mask connection interface.

The sealing cushion may be adapted to engage with a fastener portion of a mask shell. For example, the sealing cushion may be adapted to receive at least one clip or the like. In particular, the mask connection interface may comprise a recess adapted to receive enlarged portions of a corresponding projection of a mask shell.

Thus, the sealing cushion may be connected to a mask shell which comprises a gas inlet and/or outlet. The sealing cushion may form part of a nasal mask assembly for accommodating the nose only. The sealing cushion may form part of a full-face mask assembly for accommodating the nose and mouth.

The sealing cushion may be a unitary component. The sealing cushion may be formed via a single moulding process, for example by injection moulding or the like.

Although it has been described above that some of the features may be present or applicable where the respiratory mask assembly is a nasal respiratory mask assembly, and that some of the features may be present or applicable where the respiratory mask assembly is a full-face respiratory mask assembly, it will be appreciated that the features described in relation to the nasal respiratory mask assembly may also be present in or applicable to the full-face mask assembly, and the features described in relation to the full-face respiratory mask assembly may also be present in or applicable to the nasal respiratory mask assembly.

According to a second aspect of the present disclosure there is provided a sealing cushion for a respiratory mask assembly, the sealing cushion comprising a patient interface portion having a resiliently deformable sealing membrane for engagement with a patient's face, and an aperture formed therein for receiving a nasal and/or mouth region of a patient's face, the sealing membrane comprising a plurality of regions of differing thickness, the plurality of regions comprising: a compliant nasal region for engagement with the patient's nasal bridge; nasal support regions disposed either side of the compliant nasal region for engagement with the sides of the patient's nose; cheek support regions extending from each of the nasal support regions for engagement at least with the patient's cheeks; a compliant base region extending between the cheek support regions; and a compliant inner member extending around substantially all of the aperture in the patient interface portion, wherein the sealing membrane in the compliant nasal region, the compliant base region and the compliant inner member has a lower thickness than in the cheek support regions and the nasal support regions.

It will be appreciated that the features described above in relation to the first aspect of the present disclosure may also be present in or applicable to the second aspect of the present disclosure.

According to a third aspect of the present disclosure there is provided a respiratory mask assembly comprising a sealing cushion according to the second aspect of the present disclosure.

According to a fourth aspect of the present disclosure not explicitly covered by the independent claims of this application, there is provided a respiratory mask assembly comprising a sealing cushion according to the first aspect of the present disclosure.

The respiratory mask assembly may comprise a mask shell, which may be more rigid than the sealing cushion. The mask shell may be adapted so as to define an internal cavity. The mask shell may be generally dome-shaped so as to define an internal cavity. The mask shell may have a substantially concave interior surface. The mask shell may have a substantially convex exterior surface. The internal cavity may be, and is most preferably, in direct fluid communication with an internal cavity of the sealing cushion.

The mask shell may be adapted to permanently engage the sealing cushion. Alternatively, and most preferably, the mask shell may be adapted to releasably engage the sealing cushion. The sealing cushion may fit alongside an internal surface of the mask shell.

A rear surface of the mask shell may comprise at least one projection, which may extend around a peripheral region of the mask shell. The at least one projection may be shaped and/or dimensioned so as to be received by a corresponding recess of the mask shell interface portion of the sealing cushion.

The mask shell may comprise at least one fastener portion. The at least one fastener portion may be disposed in one or more localised regions of the rear surface of the mask shell. A fastener portion may be disposed along each edge of the mask shell, but may be omitted from one or more corners of the mask shell to facilitate disengagement. The at least one fastener portion may form part of the projection to be received by a corresponding recess of the mask shell interface portion of the sealing cushion, but may have dimensions larger than those of the remainder of the projection.

The at least one fastener portion may be adapted to engage, and most preferably releasably engage, a corresponding clip receiving portion of the mask interface portion. The at least one fastener portion may be shaped to engage, and most preferably releasably engage, a corresponding clip receiving portion of the mask interface portion. The at least one fastener portion may be a clip or the like.

The mask shell may comprise an aperture adapted to engage a fluid connector, or may have a fluid connector formed integrally therewith. The sealing cushion may include a fluid connector that is arranged to extend through the aperture in the mask shell when engaged. Hence, the sealing cushion and the fluid connector may be integrally formed, for example as part of a single injection moulding process. The sealing cushion and the fluid connector may be formed as a unitary body, and/or may be formed of the same material. The fluid connector may be an elbow connector. The mask shell may comprise a connecting formation, which is adapted to connect the mask shell to a fluid connector, such that the fluid connector is in fluid communication with the aperture.

The respiratory mask assembly may comprise a fluid connector for connecting the mask assembly to the remainder of a respiratory circuit. The connector may comprise first and second limbs in fluid communication. The first and second limbs may be disposed substantially perpendicularly to each other, such that the connector has an "L-shape". The connector may be a so-called elbow connector.

The mask shell may comprise at least one fastener for connecting to headgear for the respiratory mask assembly. The at least one fastener may be adapted to permanently retain a headgear strap or the like. The at least one fastener is most preferably adapted to releasably retain a headgear strap or the like. The at least one fastener may be shaped so as to releasably retain a headgear strap or the like. The mask shell most preferably comprises a plurality of fasteners, for example one on each side of the mask shell.

The fastener may extend laterally outwardly from the mask shell. The fastener may extend laterally outwardly from a lower corner of the mask shell. Most preferably, a fastener extends laterally outwardly from each of the lower corners of the mask shell. The fastener may be integrally formed with the mask shell. The fastener may be adapted to receive a corresponding portion of a headgear strap or the like. The fastener may be shaped so as to receive a corresponding portion of a headgear strap or the like. The fastener may define one or more apertures, and hence have the form of a buckle, or may have the form of a hook. Where the fastener is a hook, the fastener may be substantially "C shaped" in form.

The mask may comprise first and second fasteners, each adapted to releasably retain a headgear strap or the like. In an alternative embodiment, a first fastener may be adapted so as to permanently retain a headgear strap or the like, and a second fastener may be adapted so as to releasably retain a headgear strap or the like.

The respiratory mask assembly may further comprise headgear for retaining the respiratory mask on a patient's face, in use. The headgear may include one or more flexible straps and at least one fastener adapted to releasably attach to a corresponding fastener of the respiratory mask. The corresponding fasteners may comprise a first fastener having a neck portion and an enlarged head portion, and a second fastener having one or more support members defining an opening for accommodating the neck portion of the first fastener, such that the enlarged head portion of the first fastener bears against the one or more support members of the second fastener.

The mask shell may comprise a forehead support formation. The forehead support formation may be integrally formed with the mask shell. The forehead support formation may extend outwardly from the mask shell. The forehead support formation may extend substantially longitudinally outwardly from the mask shell. The forehead support formation may, and most preferably, extend from a corner of the mask shell adapted to be located at the nasal bridge, in use.

The forehead support formation may be adapted to substantially conform to the forehead region of a patient. The forehead support formation may be obliquely angled relative to a longitudinal axis of the mask shell. The forehead support formation may be substantially elongate in form, eg substantially elliptical in shape.

The forehead support formation may comprise a forehead rest. The forehead rest may be adapted and/or shaped so as to bear against a forehead of a patient, in use, either directly or through an intermediate component, such as the strap of headgear attached to the forehead support formation. The forehead rest may comprise a patient interface portion. The patient interface portion may comprise a resilient and/or flexible formation. The resilient and/or flexible formation may increase the comfort of a patient.

The forehead support formation may comprise at least one aperture or hook. The at least one aperture or hook may be adapted to releasably receive a headgear strap or the like.

The forehead rest may be releasably attached to the forehead support formation. Thus, the forehead rest may be replaceable with a forehead rest of a different size, for example, thereby enabling the respiratory mask assembly to accommodate patients with varying head size and/or shape.

The forehead rest may be moveable relative to the forehead support formation between first and second configurations. The first and second configurations may differ in the thickness of the forehead rest that is presented to the forehead of a patient. Thus, the forehead rest may accommodate patients with a variety of head size.

Practicable embodiments of the disclosure will now be described, by way of example only, with reference to the accompanying drawings, of which:
Figure 1 is a front view of a full-face sealing cushion from the side of a patient interface portion of the sealing cushion;
Figure 2 is a side view of the full-face sealing cushion of Figure 1;
Figure 3 is a top view of the full-face sealing cushion of Figures 1 and 2;
Figure 4 is a front view of a nasal sealing cushion from the side of a patient interface portion of the sealing cushion;
Figure 5 is a side view of the nasal sealing cushion of Figure 4;
Figure 6 is a top view of the nasal sealing cushion of Figures 4 and 5; and
Figure 7 is a perspective view of a respiratory mask comprising the nasal sealing cushion of Figures 4-6.

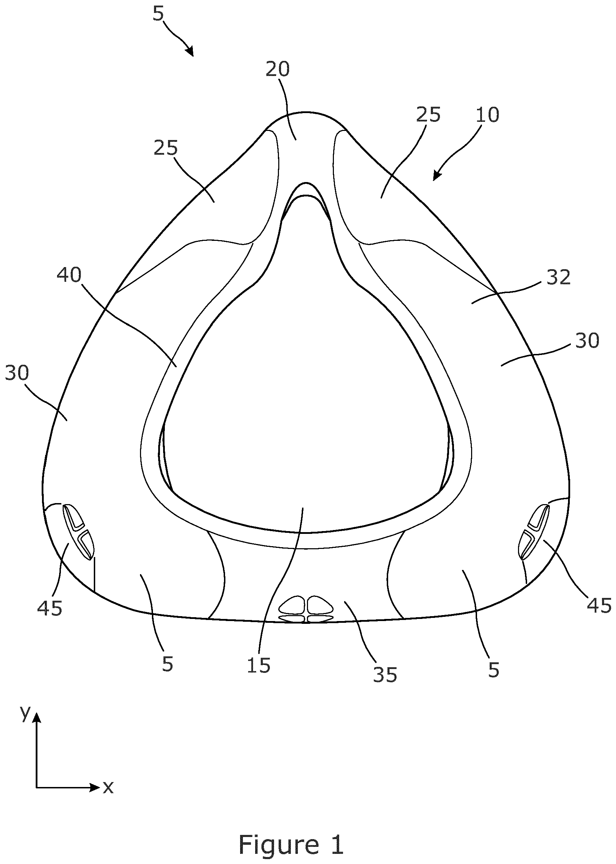

A sealing cushion according to the present technology is illustrated in Figures 1-3.

The sealing cushion 5 is substantially triangular in nature, and is shaped so as to substantially surround a patient's nasal and mouth regions and create a seal between a respiratory mask and a patient's face. The sealing cushion 5 comprises an aperture 15 therethrough for receiving both a nasal region and a mouth region of a patient. The aperture 15 has a similar shape to that of the sealing cushion 5 in general, and is thus similar to that of the nasal and mouth region of a patient.

The sealing cushion 5 comprises a patient interface portion 10, a sidewall portion 12 and a mask connection interface 55. The patient interface portion 10 and the sidewall portion 12 are formed from one continuous membrane. The sidewall portion 12 extends from the mask connection interface 55 and is folded upon itself to form the patient interface portion 10 which is arranged to engage with a person's face. Together, when in use, the patient interface portion 10, the sidewall 12 and a connected respiratory mask form an internal cavity within the sealing cushion 5.

This allows gas to be applied through the respiratory mask for the patient to inhale, and the seal formed by the patient interface portion 10 against the patient's face prevents the gas from escaping, ensuring a more efficient inhalation of the gas by the patient.

The patient interface portion 10 and the sidewall portion 12 comprise a resiliently deformable membrane that extends substantially vertically outwards from the mask connection interface 55 (ie outwards from the page as viewed in relation to Figure 1) to form the sidewall portion 12, and folds inwardly and in some places back on itself in order to create the patient interface portion 10, which is a compliant surface for engaging with a patient's face. The surface of the patient interface portion 10 is deformable upon engagement with a patient's face so as to create a sealed fit with the patient's face.

The resiliently deformable membrane varies in thickness across the patient interface portion 10 and the sidewall portion 12, creating a plurality of portions of varying thicknesses. The variation in thickness extends across the internal surface of the patient interface portion 10 and/or the internal surface of the sidewall portion 12. That is, the external surfaces of the patient interface portion 10 and the sidewall portion 12 are one continuous surface. The portions are specifically arranged so as to provide the best seal possible when the patient interface portion 10 is pressed against a patient's face during use, as well as to provide the best comfort possible to patients who can wear these masks over a long period of time.

For ease of description of the interrelation of the plurality of portions of varying thicknesses, the Figures are provided with axes, where the x axis represents the width of the sealing cushion 5 (ie cheek-to-cheek width when applied to a patient's face), the y axis represents the length or height of the sealing cushion 5 (ie nose to chin length when applied to a patient's face), and the z axis represents the depth of the sealing cushion 5 (ie outward from the face when applied to a patient's face).

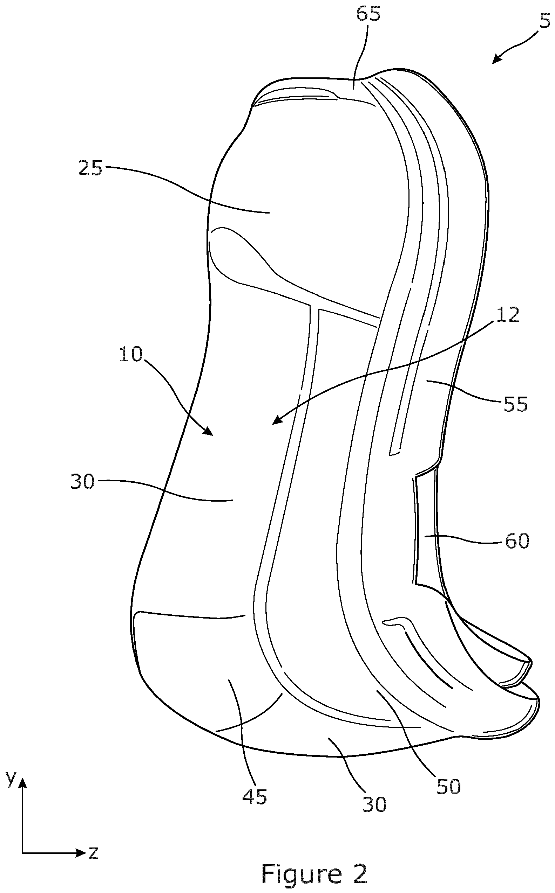

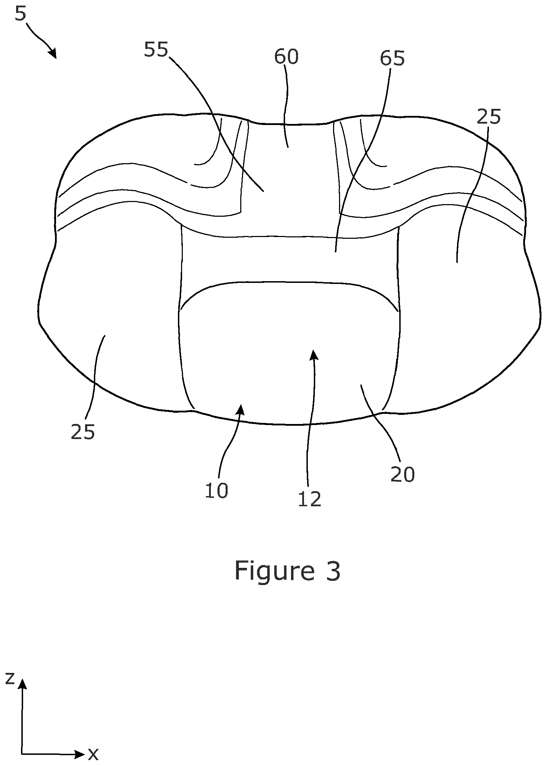

The patient interface portion 10 comprises a compliant nasal region 20, two nasal support regions 25 either side of the compliant nasal region 20, two cheek support regions 30 below each of the nasal support regions 25, a compliant chin region 35, a compliant inner member 40, two reinforced chin portions 45, and the sidewall portion 12 comprises two reinforced sidewall portions 50 and a nasal bridge support region 65 (see Figures 2 and 3).

The compliant nasal region 20 is positioned at the top of the patient interface portion and extends over the nasal bridge of the patient. The compliant nasal region 20 is a thinner portion of the patient interface portion, which allows the membrane to be particularly deformable in this area so as to comply with the patient's nose shape and size.

The nasal support regions 25 are disposed at either side of the compliant nasal region 20 and extend between the compliant nasal region 20 and the cheek support regions 30 on the patient interface portion 10. The nasal support regions 25 are thicker portions of the patient interface region which provide rigidity to the sealing cushion against the sides of the patient's nasal region, maintaining the general shape of the patient interface portion.

The cheek support regions 30 are disposed beneath each of the nasal support regions and extend between the nasal support regions 25 and the compliant chin region 35 on the patient interface portion 10. The cheek support regions are thicker than the compliant nasal region 20, but are thinner than the nasal support regions 25.

The reinforced chin portions 45 comprise additional portions formed on the internal surface of the cheek support regions 30. The reinforced chin portions 45 are disposed either side of the patient's chin region, such that they are located at the base corners of the patient interface portion 10. The reinforced chin portions 45 are of a greater thickness than the compliant nasal region 20 and the cheek support regions 30, but a lesser thickness than the nasal support regions 25.

The compliant chin region 35 is disposed between the cheek support regions 30 in the central base region of the patient interface portion 10 and is configured to engage with the patient's upper chin. The compliant chin region 35 is a thinner portion of the patient interface portion 10, having the same thickness as the compliant nasal region 20, which allows the membrane to be particularly deformable in this area so as to comply with the patient's lower mouth and jaw shape and size.

The compliant inner member 40 extends around the entirety of the perimeter of the aperture 15 in the patient interface portion 10 and merges with the compliant nasal region 20 and the compliant chin region 35. The compliant inner member 40 is a thinner portion of the patient interface portion, having the same thickness as the compliant nasal region 20 and the compliant chin region 35. This allows the patient interface portion to deform inwardly when pressed against the patient's face in use.

Figure 2 illustrates the sealing cushion 5 of Figure 1 viewed from a side angle. From this angle, the mask connection interface 55 can be seen. The mask connection interface 55 comprises a rim which extends around substantially the entire sealing cushion 5. The rim comprises a groove which extends around substantially all of the perimeter of the mask connection interface 55. The groove is dimensioned so as to receive a corresponding projection formed on the respiratory mask to be connected. The groove has portions of enlarged depth 60 located at the corners of the mask connection interface 55 ie the base corners and the apex of the sealing cushion 5. These reduced depth groove portions 60 are arranged such that corresponding outwardly protruding features formed on the respiratory mask can be received by the mask connection interface 55.

From this angle the reinforced sidewall region 50 can also be seen. The reinforced sidewall region 50 extends between the mask connection interface 55 and the cheek support region 30 in the z axis, and between the nasal support region 25 and the cheek support region 30 in the y axis. A reinforced sidewall region of the same shape, form and thickness can also be found on the opposite side of the patient interface portion 10, which cannot be seen from this view. That is, the patient interface portion 10 is completely symmetrical about a centreline in the y axis and in the y plane.

The reinforced sidewall region 50 is thicker than the reinforced chin portions 45, the cheek support regions 30, the compliant nasal region 20, the compliant chin region 35 and the compliant inner member 40, but is thinner than the nasal support regions 25.

The compliant nasal region 20 comprises a narrow band extending from the nasal bridge support region 65, approximately halfway up the sidewall portion 12 in the z direction, across the fold line between the patient interface portion 10 and the sidewall portion 12. The band begins at its widest, narrowing slightly in the x direction over the sidewall portion 12, before narrowing more dramatically between the fold line between the patient interface portion 10 and the sidewall portion 12. At the apex of the aperture 15 in the patient interface portion 10, the compliant nasal region 20 merges with the compliant inner member 40.

The nasal support regions 25 comprise lobes that extend between the compliant inner member 40, across the fold line between the patient interface portion 10 and the sidewall portion 12, to the mask connection interface 55. The entirety of each nasal support region 25, if the patient interface portion 10 and the sidewall portion 12 were laid flat, is substantially ear-shaped. That is, the portion of the nasal support region 25 on the patient interface portion 10 has an undulating edge that emulates the outer edge of an ear-shape.

Towards the top of the sealing cushion 5, nearest the compliant nasal region 20, each nasal support region 25 extends the full distance between the mask connection interface 55 and the compliant inner member. Moving away from the compliant nasal region 20 in the y direction, each nasal support region 25 reduces in width, and at its thinnest point extends only from the mask connection interface 55 to the fold line between the patient interface portion 10 and the sidewall portion 12. The diagonal line between the largest width and the smallest width of each nasal support region 25 is an undulating edge.

The cheek support regions 30 substantially follow the curvature of the sealing cushion 5 and the aperture 15. That is, on the patient interface 10, the cheek support regions 30 extend substantially downwards from the nasal support regions 25 and then follow the curvature of the corners of the sealing cushion 5 inwardly towards the compliant chin region 35. Similarly, on the sidewall portion 12, the cheek support regions 30 extend substantially downwards from the nasal support regions 25 and then follow the curvature of the corners of the sealing cushion 5 inwardly towards the compliant chin region 35 and the mask connection interface 55. The cheek support regions 30 begin approximately half way down the sidewall portion 12, and end at the bottom of the sidewall portion 12.

Towards the bottom of the sealing cushion, nearest the compliant chin region 35, each cheek support region 30 extends the full distance between the reinforced sidewall regions 50 and the compliant inner member 40. However, in an upper portion 32 of each cheek support region 30, once the reinforced sidewall portions 50 end, each cheek support region narrows in width and instead extends between the nasal support regions 25 and the compliant inner member 40. The upper portion 32 of each cheek support region 30 therefore gradually narrows in width from bottom to top in the y direction, the diagonal line between the largest width and the smallest width of the upper portion 32 comprising an undulating edge that corresponds to the undulating edge of the corresponding nasal support region 25.

The compliant chin region 35 comprises a substantially square shaped portion that extends across the fold line between the patient interface portion 10 and the sidewall portion 12. The compliant chin region 35 has a more gradual progression between the patient interface portion 10 and the sidewall portion 12 than the other regions of the sealing cushion 5, having less of a definitive fold line between the two.

The reinforced chin portions 45 comprise substantially square-shaped portions that extend across the folding line between the patient interface portion 10 and the sidewall portion 12. The reinforced chin portions have a width approximately the same as the width of the compliant nasal region 20.

The reinforced sidewall regions 50 substantially follow the curvature of the sealing cushion 5 and the aperture 15, and extend over the sidewall portion 12 substantially parallel to the cheek support region 30. That is, on the sidewall portion 12, the reinforced sidewall region 50 extend substantially downwards from the nasal support regions 25 and then follow the curvature of the corners of the sealing cushion 5 inwardly towards the mask connection interface 55. The reinforced sidewall portions 50 begin approximately halfway down the sidewall portion 12 (ie substantially in line with the start of the cheek support region 30) and end at the cheek support region 30.

The compliant inner member 40 substantially follows the curvature of the sealing cushion 5 and the aperture 15. The compliant inner member 40 has substantially the same width over its entire perimeter, aside from portions substantially adjacent the widest portions of the nasal support regions 25, which are slightly wider than the rest of the compliant inner member 40.

Figure 3 illustrates the sealing cushion 5 of Figures 1 and 2 from the top of the sealing cushion 5. From this angle, the nasal bridge support region 65 can be seen. The nasal bridge support region 65 extends between the mask connection interface 55 and the compliant nasal region 20 in the z axis, and between the two nasal support regions 25 in the x axis.

The nasal bridge support region 65 is of the same thickness as the reinforced sidewall portions 50, that is, thicker than the reinforced chin portions 45, the cheek support regions 30, the compliant nasal region 20, the compliant chin region 35 and the compliant inner member 40, but thinner than the nasal support regions 25.

The nasal bridge support region 65 is positioned so that it is unlikely to make contact with a patient's nasal bridge region in use, but so that it can provide support to the compliant nasal region 20. The nasal bridge support region 65 comprises a region having a width in the x direction greater than its length in the z direction. The width of the nasal bridge support region 65 in the x direction is substantially the same as the width in the x direction of the compliant nasal region 20. The nasal bridge support region 65 comprises concave edges in the x direction.

In this specific embodiment, each of the regions of the full-face sealing cushion 5 has the following thickness:

| | |
|---|---|
| compliant nasal region 20 | 0.35mm |
| nasal support regions 25 | 2.5mm |
| cheek support regions 30 | 1.35mm |
| compliant chin region 35 | 0.35mm |
| compliant inner member 40 | 0.35mm |
| reinforced chin portions 45 | Variable up to 1.93mm |
| reinforced sidewall regions 50 | 2.0mm |
| nasal bridge support region 65 | 2.0mm |

It will be appreciated that the thicknesses of the compliant nasal region 20, the compliant chin region 35 and the compliant inner member 40 may be the same or substantially the same, and that the thicknesses of the reinforced sidewall regions 50 and the nasal bridge support region 65 may be the same or substantially the same.

In alternative embodiments, it is anticipated that the thicknesses of each of the regions 20, 25, 30, 35, 40, 45, 50, 65 may be different, or even that the order of thicknesses of each of the regions 20, 25, 30, 35, 40, 45, 50, 65 may be different. However, the thickness of the compliant regions 20, 35, 40 will always be less than the remaining regions 25, 30, 45, 50, 65.

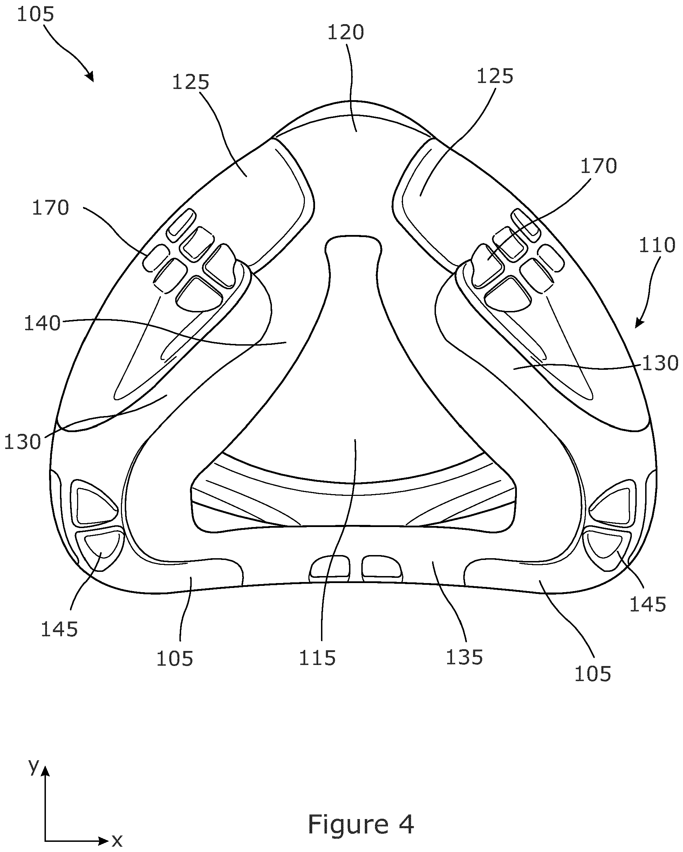

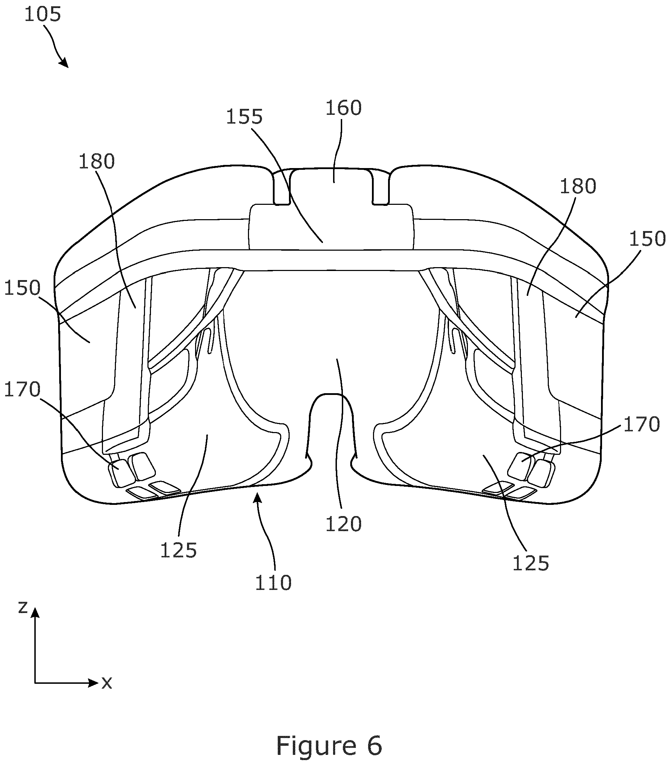

A nasal sealing cushion according to the present disclosure is illustrated in Figures 4-6.

The nasal sealing cushion 105 works in substantially the same way as the full-face sealing cushion 5 of Figures 1-3, but sealing over just the nose of a patient in use, rather than the nose and mouth of the patient, and is generally the same in nature as the sealing cushion 5 of Figures 1-3, aside from the obvious dimensional differences that result in the lower portion of the mask engaging with a patient's upper lip region in use. Corresponding features are therefore labelled with similar reference numerals, and similarities between the cushions will not be re-described for conciseness. However, features of the nasal sealing cushion 105 that differ relative to the full-face sealing cushion 5 will be described below.

For ease of description of the interrelation of the plurality of portions of varying thicknesses, the Figures are provided with axes, where the x axis represents the width of the sealing cushion 105 (ie cheek-to-cheek width when applied to a patient's face), the y axis represents the length or height of the sealing cushion 105 (ie nose to upper lip length when applied to a patient's face), and the z axis represents the depth of the sealing cushion 105 (ie outward from the face when applied to a patient's face).

The patient interface portion 100 comprises a compliant nasal region 120, two nasal support regions 125 either side of the compliant nasal region 120, two cheek support regions 130 generally below each of the nasal support regions 125, a compliant upper lip region 135, a compliant inner member 140, two reinforced upper lip portions 145, and the sidewall portion 112 comprises two reinforced sidewall portions 150. Each nasal support region 125 further comprises a reinforced nasal region 170, and each sidewall region 112 further comprises an upper reinforcing member 180 and a lower reinforcing member 190.

The nasal support regions 125 are disposed at either side of the compliant nasal region 20 and extend between the compliant nasal region 120 and a portion of the cheek support regions 130 on the patient interface portion 110.

The cheek support regions 130 are disposed generally beneath each of the nasal support regions 125 and extend between the nasal support regions 125 and the compliant upper lip region 135 on the patient interface portion 110. A portion of the cheek support regions 130 does extend adjacent to the nasal support regions 125, ie upwardly of the bottommost point of the nasal support region 125.

The reinforced upper lip portions 145 comprise additional portions formed on the internal surface of the cheek support regions 130. The reinforced upper lip portions 145 are disposed either side of the compliant upper lip region 135, such that they are located at the base corners of the nasal sealing cushion 105. The reinforced upper lip portions 145 have generally the same shape and form as the reinforced chin portions 45 of the full-face sealing cushion 5, both being reinforced subregions of the sealing cushion, but due to the dimensions of the nasal sealing cushion 105, are arranged to engage with the upper lip region of a wearer in use, rather than the chin region.

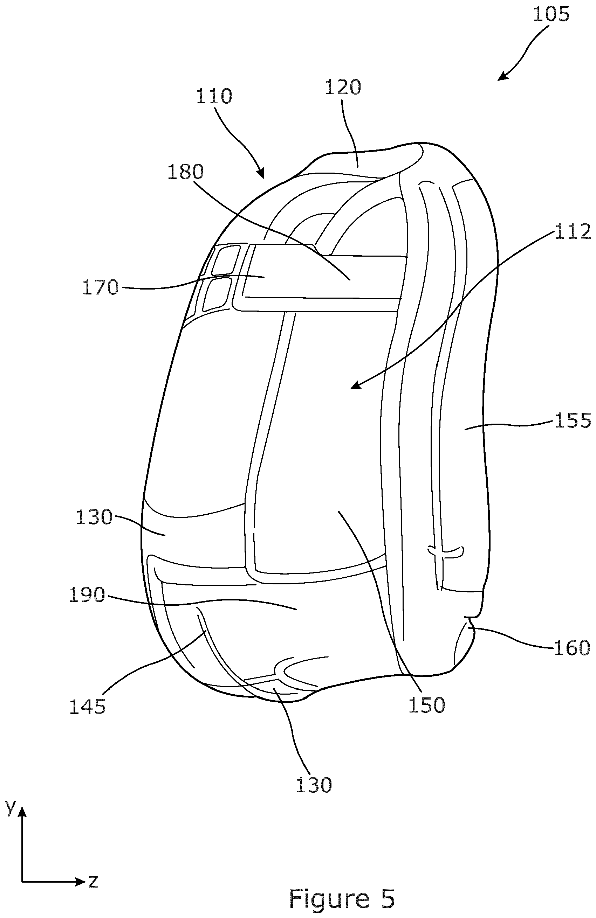

Figure 5 illustrates the sealing cushion 105 of Figure 4 viewed from a side angle. From this angle the reinforced sidewall region 150 can be seen. The reinforced sidewall region 150 extends between the mask connection interface 155 and the cheek support region 130 or the nasal support region 125 in the z axis, and between the compliant nasal region 120 and the base corner in the y axis. A reinforced sidewall region of the same shape, form and thickness can also be found on the opposite side of the nasal sealing cushion 105, which cannot be seen from this view. That is, the nasal sealing cushion 105 is completely symmetrical about a centreline in the y axis and in the y plane.

The nasal support regions 125 comprise lobes that extend from the reinforced sidewall region 150 across the fold line between the sidewall portion 112 and the patient interface portion 110, to the compliant inner member 140, or where the cheek support region 130 extends adjacent to the nasal support region 125, to the cheek support region 130

Towards the top of the sealing cushion 105, nearest the compliant nasal region 120, each nasal support region 125 extends the full distance between the reinforced sidewall region 150 and the compliant inner member 140. Moving away from the compliant nasal region 120 in the y direction, each nasal support region 125 reduces in width, and at its thinnest point extends only from the reinforced sidewall region 150 to just beyond the fold line between the patient interface portion 100 and the sidewall portion 112. The diagonal line between the largest width and the smallest width of each nasal support region 25 is a rounded edge.

From this angle, it can be seen that the upper reinforcing member 180 extends across the sidewall 112 between the reinforced nasal region 170 and the mask connection interface 155, and that the lower reinforcing member 190 extends across the sidewall 112 between the reinforced upper lip region 145 and the mask connection interface 155. In this respect, the upper reinforcing member 180 splits the reinforced sidewall region 150 into an upper section and a middle section, and the lower reinforcing member 190 splits the reinforced sidewall region 150 into a middle section and a lower section. Each of the upper reinforcing members 180 and the lower reinforcing members 190 is a generally rectangular portion having a constant width across its length. At the end of the upper reinforcing members 180 and the lower reinforcing members 190 nearest to the aperture 115, the upper reinforcing members 180 and the lower reinforcing members 190 transition into the reinforced nasal regions 170 and the reinforced upper lip regions 145 respectively.

The reinforced nasal regions 170 are positioned on the nasal support regions between the upper reinforcing members 180 and the aperture 115, and each consist of a plurality of projections that are raised relative to the underside of the nasal support regions 125, ie these portions have a greater thickness than the nasal support regions 125. In this embodiment, each reinforced nasal region 170 consists of six square-shaped projections and one larger square-shaped projection, though it will be understood that any number is possible. In this embodiment, the larger square-shaped projection is of a width and length approximately equal to two times the width and length of the smaller square-shaped projections.

The reinforced upper lip regions 145 are positioned on the cheek support regions 130 between the lower reinforcing members 190 and the aperture 115, and each consist of a plurality of projections that are raised relative to the underside of the cheek support regions 130, ie these portions have a greater thickness than the cheek support regions 130. In this embodiment, each reinforced upper lip region 145 consists of two smaller square-shaped projections and one larger square-shaped projection, though it will be understood that any number is possible. In this embodiment, the larger square-shaped projection is of a width and length approximately equal to two times the width and length of the smaller square-shaped projections

In this specific embodiment, each of the regions of the nasal sealing cushion 105 has the following thickness:

| | |
|---|---|
| compliant nasal region 120 | 0.3mm |
| nasal support regions 125 | 1.0mm |
| cheek support regions 130 | 0.7mm |
| compliant upper lip region 135 | 0.3mm |
| compliant inner member 140 | 0.3mm |
| reinforced upper lip region 145 | Variable up to 1.25mm |
| reinforced sidewall regions 150 | 2.0mm |
| reinforced nasal regions 170 | Variable up to 1.9mm |
| upper reinforcing members 180 | 2.3mm |
| lower reinforcing members 190 | 2.25mm |

It will be appreciated that the thicknesses of the reinforced sidewall regions 150 and the thickest parts of the reinforced nasal regions 170 may be substantially the same, that the thicknesses of the upper reinforcing members 180 and the lower reinforcing members 190 may be substantially the same, and that all of the compliant nasal region 120, the compliant upper lip region 135 and the compliant inner member 140 may be the same or substantially the same.

In alternative embodiments, it is anticipated that the thicknesses of each of the regions 120, 125, 130, 135, 140, 145, 150, 170, 180, 190 may be different, or even that the order of thicknesses of each of the regions 120, 125, 130, 135, 140, 145, 150, 170, 180, 190 may be different. However, the thickness of the compliant regions 120, 135, 140 will always be less than the remaining regions 125, 130, 145, 150, 170, 180, 190.

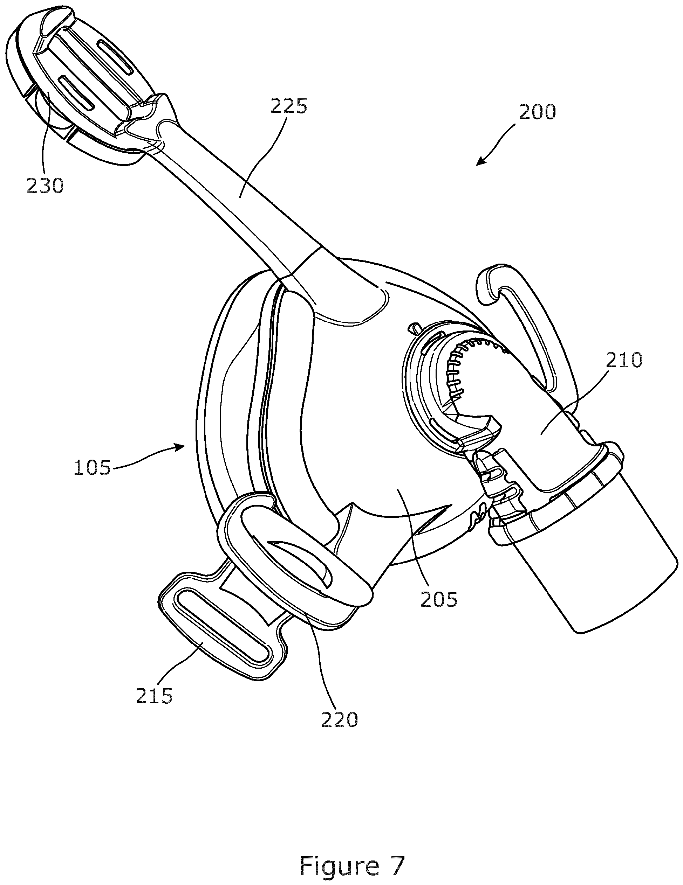

As an example, Figure 7 illustrates the nasal sealing cushion of Figures 4-6 as part of a respiratory mask assembly, generally designated 200. The mask assembly 200 comprises a mask shell 205, an elbow connector 210, and the nasal sealing cushion 105 described in relation to Figures 4-6. It will be appreciated that a similar mask shell, though of different dimensions, would engage in the same or a similar manner with the full-face sealing cushion of Figures 1-3.

The mask shell 205 is substantially triangular in nature, and is shaped so as to correspond to the mask shell interface portion 155 of the sealing cushion 105. The mask shell 205 is generally dome-shaped, such that the mask shell 205 has an internal cavity. A lip is located along each edge of the mask shell 205 so as to have a snap fit engagement with the groove of the mask connection interface 155 of the sealing cushion 105.

A pair of strap retaining formations 220 are located at lower left and right corners of the mask shell 205 and are each dimensioned so as to be received within a corresponding portion of enlarged depth of the groove disposed on the mask connection interface 155 of the sealing cushion 105. The pair of strap retaining formations 220 each extend laterally outwardly from the body of the mask shell 205. The plurality of strap retaining formations 220 are integral with the mask shell 205, and are formed as part of the same injection moulding process that is used to form the mask shell 205.

Each of the of strap retaining formations 220 comprise first and second arm-like portions. The first arm-like portion extends laterally outwardly from the mask shell 205, and the second arm like portion extends substantially orthogonally from the first arm-like portion in the same vertical plane. The second arm-like portion is curved in nature, such that a distal end of the second arm-like portion extends substantially towards the mask shell 205. Thus, each of the of strap retaining formations are hook-like in form.

A forehead support formation 225 is located at the uppermost corner of the triangularly shaped mask shell 205. The forehead support formation 225 is elongate and extends vertically outwardly from the mask shell 205. The forehead support formation 225 is integral with the mask shell 205 and is formed as part of the same injection moulding process as that which forms the mask shell 205.

The forehead support formation 225 is obliquely angled relative to the mask shell 205, so as to conform to the plane of a patient's forehead. The forehead support formation 225 has a substantially rectangular cross section. The cross section of the forehead support formation 225 is not constant along its length, and the forehead support formation 225 is tapered towards a distal end.

The distal end of the forehead support formation 225 comprises a forehead rest 230. The forehead rest 230 is integral with the forehead support formation 225 and is formed as part of the same injection moulding process. The forehead rest 230 is substantially elliptical in form and is positioned such that its semi-major axis lies in a substantially vertical direction.

The mask assembly 200 further comprises headgear, not shown in the Figures, for fastening the mask assembly 200 to the head of a patient. The headgear includes a plurality of straps which are engageable with the forehead rest 230 and the strap retaining formations 220 of the mask shell.

The headgear comprises a plurality of retaining formations 215 for engaging the corresponding strap retaining formations 220 of the mask shell 205.

The retaining formations 215 comprise a substantially rectangular main body, and end toggles, of which only one end toggle is shown in the Figures. The width of the toggles is greater than the width of the main body, such that the shape of the retaining formations 215 resembles that of a bow. An end toggle is provided with an aperture, which receives a loop of a headgear strap. The end toggle is shaped so as to correspond with and be retained by the plurality of strap retaining formations 220 of the mask shell 205.

Similarly, a loop of a strap, or two straps, of the headgear may be received within a central slot of the forehead rest 230, so as to retain the mask assembly in place relative to the face of a patient.

The elbow connector 210 comprises a lower portion and an upper portion. The lower portion is substantially cylindrical in form and configured to receive a respiratory tube for supply of oxygen or pressurised gases to the patient via the mask assembly 200. The upper portion comprises first and second perpendicular limbs which form an elbow joint. The first limb is of a significantly shorter length than the second limb, such that the upper portion of the elbow connector 210 resembles a severely truncated "L-shape" or elbow. Both the lower and upper portions of the elbow connector 210 are hollow.

The first limb of the upper portion of the elbow connector 210 is received within an aperture of the mask shell 205 with a snap fit. The elbow connector 210 is rotatable around an axis that is orthogonal to the plane of the aperture of the mask shell 205, allowing a respiratory tube to be connected from any angle.

Additional features of such a respiratory mask assembly may be found in GB25212644.

## Claims

1. A sealing cushion (5, 105) for a respiratory mask assembly, the sealing cushion comprising a patient interface portion (10, 110) having a resiliently deformable sealing membrane for engagement with a patient's face, and an aperture (15, 115) formed therein for receiving a nasal and/or mouth region of a patient's face, the sealing membrane comprising a plurality of regions of different thicknesses, the plurality of regions comprising:
a compliant nasal region (20, 120) for engagement with the patient's nasal bridge;
nasal support regions (25, 125) disposed either side of the compliant nasal region (20, 120) for engagement with the sides of the patient's nose;
cheek support regions (30, 130) extending from each of the nasal support regions (25, 125) for engagement at least with the patient's cheeks;
a compliant base region (35, 135) extending between the cheek support regions (30, 130),
and
a compliant inner member (40, 140) extending around substantially all of the aperture (15, 115) in the patient interface portion (10, 110),
wherein the sealing membrane in the compliant nasal region (20, 120), the compliant base region (35, 135) and the compliant inner member (40, 140) has a lower thickness than in the cheek support regions (30, 130) and the nasal support regions (25, 125).

2. A sealing cushion (5, 105) according to Claim 1, wherein each cheek support region (30, 130) comprises a reinforced subregion (45, 145) that is separated from the nasal support regions (25, 125).

3. A sealing cushion (5, 105) according to Claim 1 or 2, wherein the sealing cushion (5, 105) comprises a mask connection interface (55, 155) for connection to a respiratory mask body and a sidewall portion (12, 112) extending between the mask connection interface (55, 155) and the patient interface portion (10, 110).

4. A sealing cushion (5, 105) according to Claim 3, wherein the patient interface portion (10, 110) and/or the sidewall portion (12, 112) have a continuous outer surface.

5. A sealing cushion (5, 105) according to any preceding claim, wherein the thickness of each of the plurality of regions is constant across the region and the transitions between the plurality of regions are distinct step-like transitions.

6. A sealing cushion (5, 105) according to any of Claims 2-5, wherein the reinforced subregions (45, 145) are disposed at base corners of the patient interface portion (10, 110).

7. A sealing cushion (5, 105) according to any preceding claim, wherein the sealing membrane in the nasal support regions (25, 125) has a greater thickness than in the cheek support regions (30, 130).

8. A sealing cushion (5, 105) according to any of Claims 3-7, wherein the sidewall portion (12, 112) comprises reinforced sidewall regions (50, 150) disposed between the cheek support regions (30, 130) and the mask connection interface (55, 155) and/or between the nasal support regions (25, 125) and the cheek support regions (30, 130), and/or
wherein the sidewall portion (12, 112) comprises a nasal bridge support region (65, 165) disposed at an apex of the sidewall portion (12, 112), and the nasal bridge support region (65, 165) extends between the mask connection interface (55, 155) and the compliant nasal region (20, 120) and/or between the nasal support regions (25, 125).

9. A sealing cushion (5, 105) according to any preceding claim, wherein the nasal support regions (25, 125) extend along left and right sides of the patient interface portion (10, 110) between the compliant nasal region (20, 120) and the cheek support regions (30, 130), and/or
wherein the nasal support regions (25, 125) extend across at least a portion of the sidewall portion (12, 112), thereby forming a reinforced subregion of the sidewall portion (12, 112).

10. A sealing cushion (5, 105) according to any preceding claim, wherein the cheek support regions (30, 130) extend along left and right sides of the patient interface portion (10, 110) between the nasal support regions (25, 125) and the base corners of the patient interface portion (10, 110), and wherein the cheek support regions (30, 130) further extend along a base side of the patient interface portion (10, 110) between the base corners and the compliant base region (35, 135).

11. A sealing cushion (5, 105) according to any of Claims 2-10, wherein the reinforced subregions (45, 145) comprise substantially square-shaped portions, and/or
wherein the reinforced subregions (45, 145) extend from the patient interface portion (10, 110) across the sidewall portion (12, 112), and/or
wherein the reinforced subregions (45, 145) extend from the base corners of the cheek support regions (30, 130) across the sidewall portion (12, 112) to the reinforced sidewall portions.

12. A sealing cushion (5, 105) according to any preceding claim, wherein the compliant inner member (40, 140) has substantially the same width over the majority of its perimeter.

13. A sealing cushion (5, 105) according to any of Claims 3-12, wherein at least a portion of each nasal support region (25, 125) extends from the compliant inner member (40, 140) of the patient interface portion (10, 110) across the sidewall portion (12, 112) to the mask connection interface (55, 155), and/or wherein the compliant base region (35, 135) extends from the compliant inner member (40, 140) across the sidewall portion (12, 112) to the mask connection interface (55, 155).

14. A sealing cushion (5, 105) according to any of Claims 8-13, wherein the cheek support regions (30, 130) extend between the compliant inner member (40, 140) and the reinforced sidewall portions.

15. A respiratory mask assembly (200) comprising the sealing cushion (5, 105) according to any of Claims 1-14.

## Patentansprüche

1. Dichtkissen (5, 105) für eine Atemmaskenanordnung, wobei das Dichtkissen einen Patientenschnittstellenabschnitt (10, 110) mit einer elastisch verformbaren Dichtmembran zum Eingriff mit dem Gesicht eines Patienten und eine darin gebildete Öffnung (15, 115) zum Aufnehmen eines Nasen- und/oder Mundbereichs des Gesichts eines Patienten umfasst, wobei die Dichtmembran eine Vielzahl von Bereichen unterschiedlicher Dicke umfasst, wobei die Vielzahl von Bereichen Folgendes umfasst:
einen nachgiebigen Nasenbereich (20, 120) zum Eingriff mit der Nasenbrücke des Patienten;
Nasenstützbereiche (25, 125), die zu beiden Seiten des nachgiebigen Nasenbereichs (20, 120) zum Eingriff mit den Seiten der Nase des Patienten angeordnet sind;
Wangenstützbereiche (30, 130), die sich von jedem der Nasenstützbereiche (25, 125) zum Eingriff zumindest mit den Wangen des Patienten erstrecken;
einen nachgiebigen Basisbereich (35, 135), der sich zwischen den Wangenstützbereichen (30, 130) erstreckt,
und
ein nachgiebiges Innenelement (40, 140), das sich im Wesentlichen um die gesamte Öffnung (15, 115) in dem Patientenschnittstellenabschnitt (10, 110) erstreckt,
wobei die Dichtmembran in dem nachgiebigen Nasenbereich (20, 120), dem nachgiebigen Basisbereich (35, 135) und dem nachgiebigen Innenelement (40, 140) eine geringere Dicke als in den Wangenstützbereichen (30, 130) und den Nasenstützbereichen (25, 125) aufweist.

2. Dichtkissen (5, 105) gemäß Anspruch 1, wobei jeder Wangenstützbereich (30, 130) einen verstärkten Teilbereich (45, 145) umfasst, der von den Nasenstützbereichen (25, 125) getrennt ist.

3. Dichtkissen (5, 105) nach Anspruch 1 oder 2, wobei das Dichtkissen (5, 105) eine Maskenverbindungsschnittstelle (55, 155) zur Verbindung mit einem Atemmaskenkörper und einen Seitenwandabschnitt (12, 112), der sich zwischen der Maskenverbindungsschnittstelle (55, 155) und dem Patientenschnittstellenabschnitt (10, 110) erstreckt, umfasst.

4. Dichtkissen (5, 105) nach Anspruch 3, wobei der Patientenschnittstellenabschnitt (10, 110) und/oder der Seitenwandabschnitt (12, 112) eine durchgehende Außenfläche aufweisen.

5. Dichtkissen (5, 105) nach einem vorhergehenden Anspruch, wobei die Dicke von jedem aus der Vielzahl von Bereichen über den Bereich hinweg konstant ist und die Übergänge zwischen der Vielzahl von Bereichen unterschiedliche stufenartige Übergänge sind.

6. Dichtkissen (5, 105) nach einem der Ansprüche 2-5, wobei die verstärkten Teilbereiche (45, 145) an Basisecken des Patientenschnittstellenabschnittes (10, 110) angeordnet sind.

7. Dichtkissen (5, 105) nach einem vorhergehenden Anspruch, wobei die Dichtmembran in den Nasenstützbereichen (25, 125) eine größere Dicke als in den Wangenstützbereichen (30, 130) aufweist.

8. Dichtkissen (5, 105) nach einem der Ansprüche 3-7, wobei der Seitenwandabschnitt (12, 112) verstärkte Seitenwandbereiche (50, 150) umfasst, die zwischen den Wangenstützbereichen (30, 130) und der Maskenverbindungsschnittstelle (55, 155) und/oder zwischen den Nasenstützbereichen (25, 125) und den Wangenstützbereichen (30, 130) angeordnet sind, und/oder
wobei der Seitenwandabschnitt (12, 112) einen Nasenbrückenstützbereich (65, 165) umfasst, der an einer Spitze des Seitenwandabschnittes (12, 112) angeordnet ist, und sich der Nasenbrückenstützbereich (65, 165) zwischen der Maskenverbindungsschnittstelle (55, 155) und dem nachgiebigen Nasenbereich (20, 120) und/oder zwischen den Nasenstützbereichen (25, 125) erstreckt.

9. Dichtkissen (5, 105) nach einem vorhergehenden Anspruch, wobei sich die Nasenstützbereiche (25, 125) entlang linker und rechter Seiten des Patientenschnittstellenabschnittes (10, 110) zwischen dem nachgiebigen Nasenbereich (20, 120) und den Wangenstützbereichen (30, 130) erstrecken und/oder
wobei sich die Nasenstützbereiche (25, 125) über zumindest einen Abschnitt des Seitenwandabschnittes (12, 112) erstrecken, wodurch ein verstärkter Teilbereich des Seitenwandabschnittes (12, 112) gebildet wird.

10. Dichtkissen (5, 105) nach einem vorhergehenden Anspruch, wobei sich die Wangenstützbereiche (30, 130) entlang linker und rechter Seiten des Patientenschnittstellenabschnittes (10, 110) zwischen den Nasenstützbereichen (25, 125) und den Basisecken des Patientenschnittstellenabschnittes (10, 110) erstrecken, und wobei sich die Wangenstützbereiche (30, 130) ferner entlang einer Basisseite des Patientenschnittstellenabschnittes (10, 110) zwischen den Basisecken und dem nachgiebigen Basisbereich (35, 135) erstrecken.

11. Dichtkissen (5, 105) nach einem der Ansprüche 2-10, wobei die verstärkten Teilbereiche (45, 145) im Wesentlichen quadratische Abschnitte umfassen, und/oder
wobei sich die verstärkten Teilbereiche (45, 145) von dem Patientenschnittstellenabschnitt (10, 110) über den Seitenwandabschnitt (12, 112) erstrecken, und/oder
wobei sich die verstärkten Teilbereiche (45, 145) von den Basisecken der Wangenstützbereiche (30, 130) über den Seitenwandabschnitt (12, 112) zu den verstärkten Seitenwandabschnitten erstrecken.

12. Dichtkissen (5, 105) nach einem vorhergehenden Anspruch, wobei das nachgiebige Innenelement (40, 140) im Wesentlichen die gleiche Breite über den Großteil seines Umfangs aufweist.

13. Dichtkissen (5, 105) nach einem der Ansprüche 3-12, wobei sich zumindest ein Abschnitt jedes Nasenstützbereichs (25, 125) von dem nachgiebigen Innenelement (40, 140) des Patientenschnittstellenabschnittes (10, 110) über den Seitenwandabschnitt (12, 112) zu der Maskenverbindungsschnittstelle (55, 155) erstreckt und/oder wobei sich der nachgiebige Basisbereich (35, 135) von dem nachgiebigen Innenelement (40, 140) über den Seitenwandabschnitt (12, 112) zu der Maskenverbindungsschnittstelle (55, 155) erstreckt.

14. Dichtkissen (5, 105) nach einem der Ansprüche 8-13, wobei sich die Wangenstützbereiche (30, 130) zwischen dem nachgiebigen Innenelement (40, 140) und den verstärkten Seitenwandabschnitten erstrecken.

15. Atemmaskenanordnung (200), umfassend das Dichtkissen (5, 105) nach einem der Ansprüche 1-14.

## Revendications

1. Coussin d'étanchéité (5, 105) pour un ensemble masque respiratoire, le coussin d'étanchéité comprenant une partie interface de patient (10, 110) comportant une membrane d'étanchéité élastiquement déformable destinée à venir en contact avec le visage d'un patient, et une ouverture (15, 115) formée dans celle-ci pour recevoir une région nasale et/ou buccale du visage d'un patient, la membrane d'étanchéité comprenant une pluralité de régions d'épaisseurs différentes, la pluralité de régions comprenant :
une région nasale souple (20, 120) destinée à venir en contact avec le pont nasal du patient ;
des régions de support nasal (25, 125) disposées de chaque côté de la région nasale souple (20, 120), destinées à venir en contact avec les côtés du nez du patient ;
des régions de support de joue (30, 130) s'étendant de chacune des régions de support nasal (25, 125), destinées à venir en contact au moins avec les joues du patient ;
une région de base souple (35, 135) s'étendant entre les régions de support de joue (30, 130), et
un élément interne souple (40, 140) s'étendant sensiblement autour de la totalité de l'ouverture (15, 115) dans la partie interface de patient (10, 110),
ladite membrane d'étanchéité dans la région nasale souple (20, 120), ladite région de base souple (35, 135) et ledit élément interne souple (40, 140) comportant une épaisseur inférieure à celle des régions de support de joue (30, 130) et des régions de support nasal (25, 125).

2. Coussin d'étanchéité (5, 105) selon la revendication 1, chaque région de support de joue (30, 130) comprenant une sous-région renforcée (45, 145) qui est séparée des régions de support nasal (25, 125).

3. Coussin d'étanchéité (5, 105) selon la revendication 1 ou 2, ledit coussin d'étanchéité (5, 105) comprenant une interface de connexion de masque (55, 155) destinée à être connectée à un corps de masque respiratoire et une partie paroi latérale (12, 112) s'étendant entre l'interface de connexion de masque (55, 155) et la partie interface de patient (10, 110).

4. Coussin d'étanchéité (5, 105) selon la revendication 3, ladite partie interface de patient (10, 110) et/ou ladite partie paroi latérale (12, 112) comportant une surface externe continue.

5. Coussin d'étanchéité (5, 105) selon l'une quelconque des revendications précédentes, ladite épaisseur de chacune de la pluralité de régions étant constante dans toute la région et les transitions entre la pluralité de régions étant des transitions en paliers distinctes.

6. Coussin d'étanchéité (5, 105) selon l'une quelconque des revendications 2 à 5, lesdites sous-régions renforcées (45, 145) étant disposées au niveau de coins de base de la partie interface de patient (10, 110).

7. Coussin d'étanchéité (5, 105) selon l'une quelconque des revendications précédentes, ladite membrane d'étanchéité dans les régions de support nasal (25, 125) comportant une épaisseur supérieure à celle des régions de support de joue (30, 130).

8. Coussin d'étanchéité (5, 105) selon l'une quelconque des revendications 3 à 7, ladite partie paroi latérale (12, 112) comprenant des régions paroi latérale renforcées (50, 150) disposées entre les régions de support de joue (30, 130) et l'interface de connexion de masque (55, 155) et/ou entre les régions de support nasal (25, 125) et les régions de support de joue (30, 130), et/ou ladite partie paroi latérale (12, 112) comprenant une région de support de pont nasal (65, 165) disposée au niveau d'un sommet de la partie paroi latérale (12, 112), et ladite région de support de pont nasal (65, 165) s'étendant entre l'interface de connexion de masque (55, 155) et la région nasale souple (20, 120) et/ou entre les régions de support nasal (25, 125).

9. Coussin d'étanchéité (5, 105) selon l'une quelconque des revendications précédentes, lesdites régions de support nasal (25, 125) s'étendant le long de côtés gauche et droit de la partie interface de patient (10, 110) entre la région nasale souple (20, 120) et les régions de support de joue (30, 130), et/ou lesdites régions de support nasal (25, 125) s'étendant sur au moins une partie de la partie paroi latérale (12, 112), formant ainsi une sous-région renforcée de la partie paroi latérale (12, 112).

10. Coussin d'étanchéité (5, 105) selon l'une quelconque des revendications précédentes, lesdites régions de support de joue (30, 130) s'étendant le long de côtés gauche et droit de la partie interface de patient (10, 110) entre les régions de support nasal (25, 125) et les coins de base de la partie interface de patient (10, 110), et lesdites régions de support de joue (30, 130) s'étendant en outre le long d'un côté de base de la partie interface de patient (10, 110) entre les coins de base et la région de base souple (35, 135).

11. Coussin d'étanchéité (5, 105) selon l'une quelconque des revendications 2 à 10, lesdites sous-régions renforcées (45, 145) comprenant des parties sensiblement carrées, et/ou lesdites sous-régions renforcées (45, 145) s'étendant depuis la partie interface de patient (10, 110) sur la partie paroi latérale (12, 112), et/ou lesdites sous-régions renforcées (45, 145) s'étendant depuis les coins de base des régions de support de joue (30, 130) sur la partie paroi latérale (12, 112) jusqu'aux parties paroi latérale renforcées.

12. Coussin d'étanchéité (5, 105) selon l'une quelconque des revendications précédentes, ledit élément interne souple (40, 140) comportant sensiblement la même largeur sur la majeure partie de son périmètre.

13. Coussin d'étanchéité (5, 105) selon l'une quelconque des revendications 3 à 12, au moins une partie de chaque région de support nasal (25, 125) s'étendant depuis l'élément interne souple (40, 140) de la partie interface de patient (10, 110) sur la partie paroi latérale (12, 112) jusqu'à l'interface de connexion de masque (55, 155), et/ou ladite région de base souple (35, 135) s'étendant depuis l'élément interne souple (40, 140) sur la partie paroi latérale (12, 112) jusqu'à l'interface de connexion de masque (55, 155).

14. Coussin d'étanchéité (5, 105) selon l'une quelconque des revendications 8 à 13, lesdites régions de support de joue (30, 130) s'étendant entre l'élément interne souple (40, 140) et les parties paroi latérale renforcées.

15. Ensemble masque respiratoire (200) comprenant le coussin d'étanchéité (5, 105) selon l'une quelconque des revendications 1 à 14.
